Europäisches Patentamt

European Patent Office

Office européen des brevets

(11) Veröffentlichungsnummer: **0 291 562 B1**

(12) **EUROPÄISCHE PATENTSCHRIFT**

(45) Veröffentlichungstag der Patentschrift: 18.09.91

(51) Int. Cl.⁵: **A61F 2/34**

(21) Anmeldenummer: 87109832.3

(22) Anmeldetag: 08.07.87

(54) Aussenschale für eine künstliche Hüftgelenkspfanne.

(30) Priorität: 18.05.87 CH 1907/87

(43) Veröffentlichungstag der Anmeldung:
23.11.88 Patentblatt 88/47

(45) Bekanntmachung des Hinweises auf die
Patenterteilung:
18.09.91 Patentblatt 91/38

(84) Benannte Vertragsstaaten:
AT DE FR GB IT

(56) Entgegenhaltungen:
DE-B- 2 527 865
FR-A- 2 548 012

(73) Patentinhaber: GEBRÜDER SULZER AKTIENGE-
SELLSCHAFT
Zürcherstrasse 9
CH-8401 Winterthur(CH)

(72) Erfinder: Zweymüller, Karl, Dr.-Med-
Orthopädische Universitäts-Klinik
Garnisonstrasse 13 A-1090 Wien(AT)

Rank Xerox (UK) Business Services

## Beschreibung

Die Erfindung betrifft eine Aussenschale für eine künstliche Hüftgelenkspfanne, welche Aussenschale auf ihrer mit einer Struktur versehenen Aussenfläche ein selbstschneidendes Gewinde zur zementfreien Verankerung im Beckenknochen trägt, wobei die Umhüllenden für die Gewindespitzen unterschiedliche Winkel mit der Rotationssymmetrie-Achse der Schale bilden.

Eine Aussenschale für eine Hüftgelenkspfanne mit den vorstehend genannten Merkmalen ist bekannt aus der DE-A-36 02 081; die Umhüllenden für die Gewindespitzen verlaufenn bei dieser Konstruktion konisch zur Rotationssymmetrie-Achse der Schale, wobei sie gegebenenfalls zwei Konen mit unterschiedlichen Oeffnungswinkeln bilden. Die konische Form der Umhüllenden, bei denen der lotrechte Abstand zwischen Umhüllender und äusserer Oberfläche der Aussenschale zum Pol der Schale hin kleiner wird, soll den Einschraubvorgang erleichtern.

Schneiden sich selbstschneidende Gewinde zur zementfreien Verankerung von künstlichen Hüftgelenkspfannen in den Beckenknochen, so ergibt sich aufgrund der anatomischen Gegebenheiten die grundlegende Schwierigkeit, dass die polnahen Gewindegänge in relativ weiches spongioses Gewebe einschneiden, während die äquatornahen in harte Kortikalis eindringen müssen. Ein weiteres Problem ist die Gefahr des Verkantens beim Ansetzen und Eindrehen der Pfanne in den Knochen.

Aufgabe der Erfindung ist es daher, eine Aussenschale der eingangs genannten Art zu schaffen, die den erwähnten anatomischen Gegenbenheiten Rechnung trägt und bei der die Gefahr des Verkantens beim Ansetzen an die künstlich geschaffene Ausnehmung im Beckenknochen vermindert ist.

Diese Aufgabe wird erfindungsgemäss dadurch gelöst, dass vom polseitigen Ende des Gewindes aud für mindestens zwei vollständige Gewindegänge die Umhüllende der Gewindespitzen parallel zur Aussenfläche der Schale verläuft, und dass bei den anschliessenden distalen Gewindegängen der lotrechte Abstand zwischen Aussenfläche und Umhüllender sich kontinuierlich in distaler Richtung verringert.

Ein Verkanten, besonders einer konischen Schale beim Ansetzen zu Beginn des "Gewindeschneidens" wird dadurch verhindert dass die Schale beim Ansetzen durch die beiden ersten polnahen Gewindegängen geführt ist. Diese mit realtiv "tiefen" Gewindeflanken versehenen Gewindegängen dringen weit und relativ leicht in die weiche Spongiosa ein. Die "tiefen" Gewindeflanken haben relativ grosse "Auflagefläche" und ergeben dadurch zusätzlich eine besonders gute Haftung in axialer Richtung der Pfanne. Das relativ kurze Gewinde im äquatorialen Bereich schneidet sich ebenfalls leicht in die harte Kortikalis ein; so werden Kräfte vermieden, die zu einem "Sprengen" des Knochens führen können.

Im folgenden wird die Erfindung anhand von zwei Ausführungsbeispielen im Zusammenhang mit der Zeichnung näher erläutert.

Fig. 1    zeigt einen vergrösserten Meridian-Schnitt durch eine Hüftgelenkspfanne mit einer konischen Grundform, während

Fig. 2    in der gleichen Darstellung eine in ihrer Grundfrom halbkugelförmige Pfanne wiedergibt.

Die neue Prothese 1 für einen Hüftgelenkspfanne besteht aus einer metallenen Aussenschale 2 und einem Pfannenkörper 3 aus Kunststoff. In diesem Pfannenkörper ist die eigentliche Pfannenschale 4 vorgesehen, die zur Aufnahme eines nicht gezeigten künstlichen oder natürlichen Hüftgelenkkopfes bestimmt ist. Die Aussenschale 2 ist für eine zementfreie Verankerung im ebenfalls nicht gezeigten Beckenknochen auf ihrer äusseren Ober- oder Aussenfläche S in bekannter Weise mit einem selbstschneidenden Gewinde versehen. Gemäss der vorliegenden Erfindung ist der lotrechte Abstand a zwischen den Umhüllenden U1 und U2 für die Gewindespitzen und der äusseren Oberfläche S im polnahen Bereich verschieden zu demjenigen im äquatorialen Gewindebereich.

Im polnahen Bereich verläuft die Umhüllende U1 über eine Höhe 2P von zwei vollständigen Gewindegängen P parallel zur Aussenfläche S der Schale 2 in einem Abstand a. Daran anschliessend verringert sich der Abstand a nach distal zum Aequator 6 der Pfanne in kontinuierlich, so dass die "Tiefe" der Gewindeflanken in diesem Bereich sich stetig verkleinert.

Die Umhüllenden U1 und U2 für eine Konus-Aussenschale 2 sind mit der Aussenschale 2 konzentrische Konen, von denen der eine den gleichen Konuswinkel wie die Aussenfläche S der Schale 2 hat, während der Konuswinkel des anderen die distale Umhüllende U2 bildenden Konus flacher oder kleiner ist.

Für eine in der Grundform halbkugelförmige Aussenschale 2 wird die polnahe Umhüllende U1 erzeigt durch einen mit dem erzeugenden Kreis für die Aussenfläche S konzentrischen Kreis mit dem Radius R, der - ebenso wie der erzeugende Kreis für die Aussenfläche S - um die Rotationsachse 7 der Aussenschale 2 "rotiert".

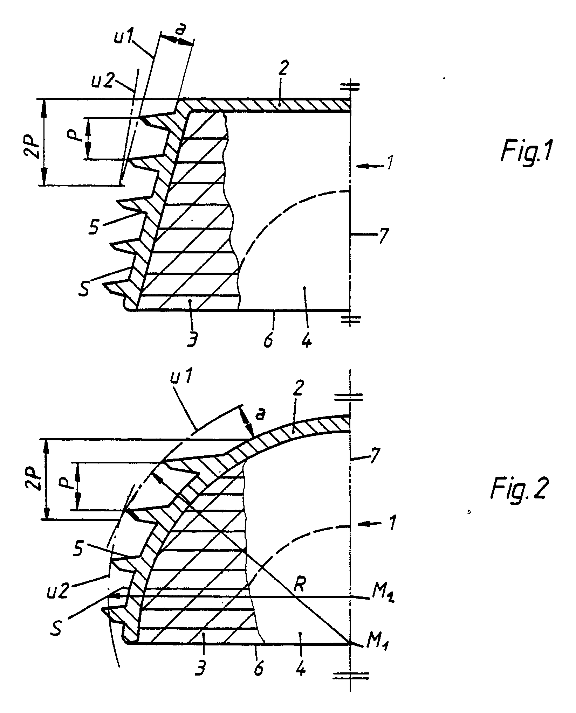

Die Umhüllenden U2 der distalen Gewindespitzen der Aussenschale 2 der Halbkugel-Pfanne ist ebenfalls ein Kreisbogen mit dem Radius R, dessen Mittelpunkt M2 jedoch gegenüber demjenigen M1 der Umhüllenden U1 in Richtung Pol verschoben und zur Achse 7 in Fig. 2 nach rechts versetzt gelegen ist.

## Patentansprüche

1.  Aussenschale für eine künstliche Hüftgelenkspfanne, welche Aussenschale (2) auf ihrer mit einer Struktur versehenen Aussenfläche (5) ein selbstschneidendes Gewinde (5) zur zementfreien Verankerung im Beckenknochen trägt, wobei die Umhüllenden (U1, U2) für die Gewindespitzen unterschiedliche Winkel mit der Rotationssymmetrie-Achse (7) der Schale bilden, dadurch gekennzeichnet, dass vom polseitigen Ende des Gewindes (5) aus für mindestens zwei vollständige Gewindegänge (P) die Umhüllende (U1) der Gewindespitzen parallel zur Aussenfläche (S) der Schale (2) verläuft, und das bei den anschliessenden distalen Gewindegängen der lotrechte Abstand (a) zwischen Aussenfläche (S) und Umhüllender (U2) sich kontinuierlich in distaler Richtung verringert.

2.  Aussenschale nach Anspruch 1, dadurch gekennzeichnet, dass sie als Zylinderkonus augebildet ist.

## Claims

1.  An outer shell for an acetabular prosthesis, which outer shell (2) has on its structured outside surface (S) a self-tapping screwthread (5) for uncemented fixing in the pelvis, the envelopes (U1, U2) for the screwthread crests forming different angles with the axis (7) of rotational symmetry of the shell, characterised in that from the polar end of the screwthread (5) the envelope (U1) of the thread crests extends for at least two complete pitches (P) parallel to the shell outside surface (S), and in the subsequent distal thread pitches the vertical distance (a) between the outside surface (S) and the envelope (U2) decreases continuously in the distal direction.

2.  An outer shell according to claim 1, characterised in that it is in the form of a cylindrical cone.

## Revendications

1.  Coque extérieure pour une cavité cotyloïde artificielle, laquelle coque extérieure (2) porte sur sa surface extérieure (S), pourvue d'une structure, un filetage (5) autotaraudeur pour l'ancrage sans ciment dans l'os iliaque, les enveloppantes (U1, U2) des pointes des filets formant des angles différents avec l'axe de symétrie de rotation (7) de la coque, caractérisée en ce qu'à partir de l'extrémité polaire du filetage (5), l'enveloppante (U1) des pointes des filets s'étend parallèlement à la surface extérieure (S) de la coque (2) pour au moins deux filets complets (P), et en ce que pour les filets distaux adjacents, la distance perpendiculaire (a) diminue en continu, dans la direction distale, entre la surface extérieure (S) et l'enveloppante (U2).

2.  Coque extérieure selon la revendication 1, caractérisée en ce qu'elle a une forme cylindro-conique.

Fig.1

Fig.2